(19) Europäisches Patentamt
European Patent Office
Office européen des brevets

(11) EP 0 966 953 B1

(12) **FASCICULE DE BREVET EUROPEEN**

(45) Date de publication et mention de la délivrance du brevet:
**17.09.2003 Bulletin 2003/38**

(51) Int Cl.[7]: **A61K 7/42**, A61K 7/00

(21) Numéro de dépôt: **99401355.5**

(22) Date de dépôt: **04.06.1999**

(54) **Compositions comprenant des nanopigments d'oxydes de fer pour la coloration artificielle de la peau et utilisations**

Eisenoxidnanopigmente enthaltende Zusammensetzungen für die künstliche Bräunung der Haut und andere Verwendungen

Compositions comprising nanopigments of iron oxides for artificial skin tanning and other uses

(84) Etats contractants désignés:
**AT BE CH CY DE DK ES FI FR GB GR IE IT LI LU MC NL PT SE**

(30) Priorité: **26.06.1998 FR 9808164**

(43) Date de publication de la demande:
**29.12.1999 Bulletin 1999/52**

(73) Titulaire: **L'OREAL**
**75008 Paris (FR)**

(72) Inventeur: **Candau, Didier**
**91570 Bièvres (FR)**

(74) Mandataire: **Dossmann, Gérard**
**Bureau D.A. Casalonga-Josse**
**Paul-Heyse-Strasse 33**
**80336 München (DE)**

(56) Documents cités:
**EP-A- 0 669 125**
**WO-A-93/11742**
**WO-A-94/21221**
**FR-A- 2 703 247**
**FR-A- 2 746 301**
**US-A- 5 064 644**
**US-A- 5 547 658**
**US-A- 5 741 480**

- **SEIFEN, OLE, FETTE, WACHSE, vol. 98, no. 16, 1972, page 512 XP002101579 AUGSBURG DE**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 966 953 B1

Printed by Jouve, 75001 PARIS (FR)

## Description

La présente invention se rapporte à de nouvelles compositions cosmétiques et/ou dermatologiques comprenant au moins un nanopigment d'oxyde de fer, pour conférer à la peau une coloration artificielle proche du bronzage naturel et à leurs utilisations dans le domaine cosmétique susmentionné.

De nos jours, de nombreux consommateurs souhaitent conserver une bonne mine et recherchent une peau bronzée. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Cependant, l'utilisation de la DHA peut présenter certains inconvénients. Ainsi, la DHA présente une fâcheuse tendance, plus ou moins prononcée selon la nature du milieu dans lequel elle est formulée, à se dégrader au cours du temps, cette dégradation se traduisant généralement à terme par un jaunissement non souhaitable des compositions qui la contiennent. Un tel phénomène fait que l'activité de la DHA, et en particulier son aptitude à colorer la peau, peut être diminuée au moment de l'application de ces compositions sur la peau. Ainsi, l'intensité de la coloration obtenue sur la peau peut apparaître comme encore insuffisante.

Un autre inconvénient de la DHA est la lenteur avec laquelle la coloration se développe : il faut en effet compter plusieurs heures (3 à 5 heures en général) pour que soit révélée la coloration. De plus, la coloration produite sur la peau par la DHA est souvent jugée trop jaune par les utilisateurs.

En vue de répondre à ce besoin, on a proposé d'associer la DHA à divers composés : ainsi, le document WO 95/15742 décrit l'association de la DHA avec des acides aminés. Cependant, l'utilisation de telles associations est très peu pratique dans la mesure où elle nécessite soit une application en deux temps soit des conditionnements séparés complexes. Le document FR-2726761 décrit quant à lui l'association de la DHA avec la lawsone et/ou la juglone : là encore cette association est peu satisfaisante, du fait cette fois des risques de sensibilisation qu'elle présente.

Ainsi, on est toujours à la recherche de nouveaux composés et de nouvelles compositions permettant de conférer artificiellement à la peau une coloration proche du bronzage naturel d'une manière simple, efficace, rapide et sans risque.

Les pigments d'oxydes de fer sont connus depuis longtemps pour leurs propriétés couvrantes et colorantes superficielles et momentanées sur la peau. Ils sont généralement utilisés dans des produits de maquillage tels que des fonds de teint, des mascaras, des eye-liners, des rouges à lèvres. Leur taille moyenne peut varier en général entre 0,2 et 50 μm en fonction des effets de coloration recherchées et de l'application envisagée. On peut citer, par exemple, les compositions de maquillage les contenant telles que décrites dans les demandes de brevet WO94/15580, WO96/36309, WO96/33690, WO96/36323, FR-A-2754708.

Les nanoparticules d'oxyde de fer (que l'on nommera ci-après nanopigments d'oxyde de fer), de taille moyenne inférieure à 200nm sont généralement utilisées pour leurs bonnes propriétés filtrantes des radiations UV dans un large spectre dans la formulation de compositions cosmétiques comme celles décrites dans les demandes de brevet FR-A-2746301 et FR-A-2746301. Elles sont également présentes dans des produits de protection solaire à base de nanopigments d'oxyde de titane ou de zinc pour atténuer et masquer la couleur bleue issue du photobleuissement sous l'action des radiations UV, comme le montre la demande de brevet WO93/11742.

A la suite d'importantes recherches menées dans le domaine de la coloration artificielle de la peau, la Demanderesse a maintenant découvert que l'utilisation de nanopigments d'oxyde de fer dans un support émulsion eau-dans-huile permettait de conférer à la peau, quelques minutes après l'application sur celle-ci, une coloration artificielle proche du bronzage naturel, à la fois intense, non-couvrante (à savoir n'ayant pas tendance à opacifier la peau) et transparente. Les colorations obtenues avec ces émulsions particulières sont de plus persistantes dans le temps et particulièrement résistantes à l'eau.

La présente invention a donc pour objet une nouvelle composition cosmétique et/ou dermatologique, destinée à produire sur la peau une coloration artificielle proche du bronzage naturel, caractérisée par le fait qu'elle comprend, dans un support sous forme d'émulsion eau-dans-huile, plus de 2% en poids par rapport au poids de ladite composition d'au moins un nanopigment d'oxyde de fer dont la taille moyenne des particules élémentaires est inférieure à 100nm

Au sens de la présente invention, on entendra, par «composition destinée à la coloration artificielle de la peau», une formulation ayant une affinité particulière pour la peau lui permettant de conférer à cette dernière une

coloration durable qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un produit de maquillage.

D'autres caractéristiques, aspects et avantages de la présente invention apparaîtront à la lecture de la description détaillée qui va suivre.

Les nanopigments d'oxyde de fer utilisés selon l'invention ont en général une taille moyenne de particules élémentaires allant de préférence de 5 nm à 50 nm, et plus particulièrement de 10 à 30 nm. Ils sont présents dans les compositions de l'invention dans des proportions supérieures à 2% en poids et de préférence allant jusqu'à 10% en poids et plus particulièrement variant de 3 à 6% en poids par rapport au poids total de la composition.

Parmi les nanopigments d'oxyde de fer utilisables, on peut citer:

- l'oxyde de fer jaune transparent (aiguilles de 10nmX100nm) vendu sous le nom « Cappoxyt jaune 4214 X » par la Société Capelle;
- l'oxyde de fer rouge transparent (aiguilles de 10nmX100nm) vendu sous le nom « Cappoxyt jaune 4435 B » par la Société Capelle;
- l'oxyde de fer jaune enrobé stéarine (10nm) vendu sous le nom « oxyde de fer transparent » par la BASF;
- l'oxyde de fer jaune micronisé vendu sous le nom « TY-220 » par la Société MITSUBISHI;
- les oxydes de fer rouges du type $\alpha$ (3nm) vendus sous les références « Nanocat superfine iron oxide » et « Nanocat SFIO rouge dispersion in minéral oil » par la Société MACH 1 ;
- l'oxyde de fer brun non enrobé (26nm) vendu sous le nom «Nanoguard WCD 2002 » par la Société NANOPHASE TECHNOLOGIES ;
- l'oxyde de fer rouge non enrobé (26nm) vendu sous le nom «Nanoguard WCD 2006 » par la Société NANOPHASE TECHNOLOGIES ;
- l'oxyde de fer noir en dispersion aqueuse à 50% (23nm) vendu sous le nom «Nanoguard Iron FE45BL AQ» par la Société NANOPHASE TECHNOLOGIES ;
- l'oxyde de fer rouge enrobé en dispersion siliconée à 40% dans le DC345 (31nm) vendu sous le nom «Nanoguard WCD 2015 » par la Société NANOPHASE TECHNOLOGIES ;
- l'oxyde de fer noir (23nm) vendu sous le nom «Nanoguard FE45BL » par la Société NANOPHASE TECHNOLOGIES ;
- l'oxyde de fer brun en dispersion siliconée à 40% dans le DC556 (26nm) vendu sous le nom «Nanoguard WCD 2008 » par la Société NANOPHASE TECHNOLOGIES ;
- l'oxyde de fer brun en dispersion à 40% dans le FINSOLV TN (26nm) vendu sous le nom «Nanoguard WCD 2009 » par la Société NANOPHASE TECHNOLOGIES ;
- l'oxyde de fer rouge en dispersion aqueuse à 50% (23nm) vendu sous le nom «Nanoguard Iron FE45R AQ» par la Société NANOPHASE TECHNOLOGIES.

Les compositions selon l'invention sont sous forme d'émulsion eau-dans-huile et comprennent généralement une phase huileuse continue qui peut comporter un ou plusieurs corps gras, ces corps gras pouvant être constitués par une huile ou une cire ou leurs mélanges.

Par huile, on entend un composé liquide à température ambiante. Par cire, on entend un composé solide ou substantiellement solide à température ambiante, et dont le point de fusion est généralement supérieur à 35°C.

Comme huiles utilisables selon l'invention, on peut citer les huiles d'origine végétale ou animale, minérale ou synthétique ; les huiles fluorées ; les triglycérides d'acides gras en $C_{12}$-$C_{18}$; les huile de silicones volatiles ou non et leurs mélanges.

Parmi les huiles d'origine végétale ou animale, modifiées ou non, on peut citer par exemple l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau (d'abricot), l'huile de calophyllum.

Parmi les huiles d'origine minérale, on peut citer par exemple l'huile de paraffine.

Parmi les huiles synthétiques, on peut citer notamment les isoparaffines volatiles ou non et les poly-isobutènes.

Comme cires utilisables dans l'invention, on peut citer les cires d'origine animale comme la lanoline, la cire d'abeilles, le spermaceti, les dérivés de la lanoline tels que les alcools de lanoline, la lanoline hydrogénée, hydroxylée ou acétylée, les acides gras de la lanoline et l'alcool de lanoline acétylée ; les cires d'origine végétale telles que la cire de Carnauba, de Candellila, de kapok, d'Ouricury, de riz, de jojoba hydrogénée, d'Alfa, du Japon ou les cires de fibres de liège ou de canne à sucre ou encore le beurre de cacao ; les cires minérales par exemple de paraffine, de montan, de lignite, de pétrolatum, de vaseline ou les cires microcristallines, la cérésine, l'ozokérite ; les cires synthétiques

comme les cires de polyéthylène, les cires obtenues par synthèse de Fischer-Tropsch et les esters linéaires résultant de la réaction d'un acide carboxylique saturé en $C_{10}$ à $C_{40}$ et d'un alcool saturé en $C_{10}$ à $C_{40}$ comme le myristate de myristyle. On peut aussi utiliser l'alcool cétylique, l'alcool stéarylique, les lanolates ou stéarates de calcium, l'huile de ricin, de palme, de coco, de tournesol ou de coprah hydrogénée.

Comme huiles de silicone utilisables selon l'invention, on peut citer les polydiorganosiloxanes linéaires, éventuellement fonctionnalisés, ou cycliques ou les organopolysiloxanes éventuellement réticulé ou les mélanges de ceux-ci.

Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en $C_1$-$C_{10}$ et en particulier méthyle, les radicaux fluoroalkyles, les radicaux aryles et en particulier phényle. Ils peuvent être par exemple substitués par des groupements ester ou éther en $C_1$-$C_{40}$, des groupements aralkyles en $C_7$-$C_{60}$.

On peut utiliser, comme huiles de silicone, des polydiorganosiloxanes linéaires éventuellement fonctionnalisés utilisables selon l'invention répondent à la formule générale suivante :

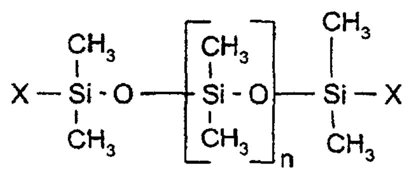

dans laquelle:

- X est $-CH_3$ ou OH, et
- n est un entier allant de 0 à 2000.

Parmi ceux-ci, on citera notamment les produits vendus sous la dénomination de "AK" par la Société WACKER, "SF" par la Société GENERAL ELECTRIC et "ABIL" par la Société GOLDSCHMIDT, tel que le produit "Abil 10".

Comme polydiorganosiloxanes cycliques selon l'invention, on peut utiliser, seuls ou en mélange, des cyclométhicones de formule:

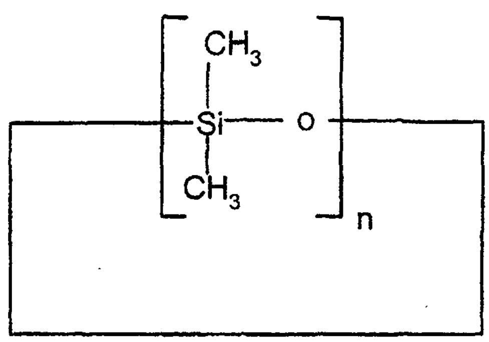

dans laquelle:

- n est un nombre entier de 3 à 8.

Parmi les cyclométhicones particulièrement préférées, on citera le cyclotétradiméthylsiloxane (n = 4), le cyclopentadiméthylsiloxane (n = 5), et le cyclohexadiméthylsiloxane (n = 6).

On peut notamment utiliser les produits vendus sous les dénominations de "DC Fluid 244", "DC Fluid 245", "DC FLuid 344 et "DC Fluid 345" par la Société Dow Corning.

D'autres cyclométhicones utilisables selon l'invention sont celles vendues sous les dénominations de "Abil K4" par la Société GOLDSCHMIDT ; sous les dénominations de "Silbione 70045 V2" et de "Silbione Huile 70045 V5" par la Société RHONE POULENC; ainsi que sous les dénominations de "Volatil Silicone 7158" et de "Volatil Silicone 7207" par la Société UNION CARBIDE.

Les compositions de l'invention peuvent également comprendre d'autres composés siliconés. Comme les polyalkyl($C_1$-$C_{20}$)siloxanes, dont les huiles de silicone phénylées, ainsi que les gommes de silicones et les cires de silicone.

Les gommes de silicone utilisables dans la composition de l'invention peuvent être des polysiloxanes de masse moléculaire élevée, de l'ordre de 200 000 à 1 000 000 et de viscosité supérieure à 500 000 mPa.s. Elles peuvent être utilisées seules ou en mélange avec un solvant tel qu'une huile polydiméthylsiloxane ou polyphénylsiloxane, ou une cyclométhicone.

Les cires de silicone utilisables dans la composition selon l'invention peuvent être des polysiloxanes linéaires substitués. On peut citer, par exemple, les cires de silicone polyéther, les alkyl ou alkoxy-diméthicones ayant de 16 à 45 atomes de carbone.

Les compositions selon l'invention peuvent également comprendre des résines de silicone comprenant une combinaison des unités $R_3SiO_{½}$, $R_2SiO_{2/2}$ $RSiO_{3/2}$ et $SiO_{4/2}$.

Selon une forme particulièrement préférée de l'invention, les compositions se présentent sous forme d'émulsion eau-dans-silicone dans laquelle la phase huileuse continue comprend au moins une huile de silicone telle que définie ci-dessus. On utilisera plus particulièrement des huiles de silicone volatiles, tels que les cyclométhicones, pour obtenir une plus grande rémanence à l'eau de la coloration sur la peau, un étalement plus facile et plus homogène de la composition ainsi que des temps de séchage plus courts.

Lorsque les compositions de l'invention se présentent sous forme d'émulsion eau-dans-silicone, les huiles de silicone utilisées selon l'invention sont de préférence présentes en une proportion d'au moins 5 % et de préférence allant de 10 à 45 % en poids par rapport au poids total de l'émulsion. La phase grasse de l'émulsion eau-dans-huile selon l'invention peut comprendre en plus une ou des huile(s) hydrocarbonée(s) dans une proportion allant de préférence jusqu'à 40 % en poids par rapport au poids total de la phase grasse de l'émulsion.

Les compositions conformes à l'invention comprennent de préférence un émulsionnant siliconé constitué d'un polyalkyl polyéther siloxane portant des chaînes polyoxyéthylènes et polyoxypropylènes greffées sur la chaîne principale ou aux extrémités de la chaîne principale..

Cet émulsionnant siliconé peut être choisi parmi les composés de formule générale (I) suivante :

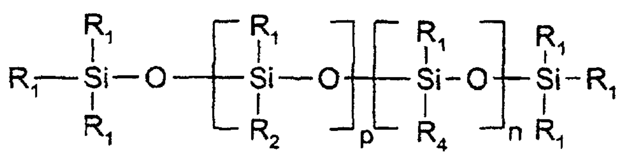

(I)

formule dans laquelle

- $R_1$ et $R_4$, identiques ou différents, représentent un atome d'hydrogène, un radical alkyle linéaire ou ramifié en $C_1$-$C_{30}$ ou un radical phényle,
- $R_2$, identiques ou différents, représentent $-(C_xH_{2x})-(OC_2H_4)_a-(OC_3H_6)_b-OR_3$,
- $R_3$, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 1 à 1000,
- p varie de 1 à 30,
- a varie de 1 à 50,
- b varie de 1 à 50,
- x varie de 1 à 5.

Le poids moléculaire moyen en nombre de cet émulsionnant siliconé est en général supérieur ou égal à 15000 et de préférence compris entre 20 000 et 40 000.

Une première famille de polyalkyl polyéther siloxanes convenant particulièrement bien aux compositions selon l'invention est celle des composés répondant à la formule (I) ci-dessus pour lesquels les radicaux $R_1$ et $R_4$ sont identiques et représentent tous des radicaux méthyle et le radical $R_3$ représente l'hydrogène.

A titre d'exemple d'émulsionnant siliconé appartenant à cette famille, on peut citer le poly diméthyl/méthyl siloxane oxyéthyléné oxypropyléné (OE/OP 18/18) pour lequel n est 396 et p est 4, de poids moléculaire en nombre

supérieur à 30 000 (nom CTFA : Cyclométhicone 90% Dimethicone copolyol 10%) vendu sous la dénomination commerciale de « Silicone Q2-3225C » par la société Dow Coming.

Dans la définition ci-dessus et dans la suite du texte, OE représente une mole d'oxyde d'éthylène et OP représente une mole d'oxyde de propylène.

Une deuxième famille de polyalkyl polyéther siloxanes convenant particulièrement bien aux compositions selon l'invention est celle des composés répondant à la formule (I) ci-dessus pour lesquels les radicaux $R_1$ représentent tous des radicaux méthyle et les radicaux $R_4$ représentent tous des radicaux lauryle.

Un émulsionnant siliconé particulièrement préféré de cette deuxième famille est le poly méthyllauryl/méthyl siloxane oxyéthyléné oxypropyléné (OE/OP 18/18) pour lequel n est 35 et p est 3, de poids moléculaire en nombre supérieur à 25 000 (nom CTFA: Laurylméthicone copolyol 91%, Isostéaryl alcohol 9%) vendu sous la dénomination commerciale « DC Q2-5200 » par la société Dow Coming.

Un émulsionnant siliconé tout particulièrement préféré pour les compositions selon l'invention est une silicone oxyalkyléne substituée en α-ω à structure linéaire, substitué aux deux extrémités de la chaîne principale par des groupements oxyalkylènes reliés aux atomes de Si par l'intermédiaire d'un groupement hydrocarboné. On choisira plus particulièrement une silicone répondant à la formule générale (II) suivante :

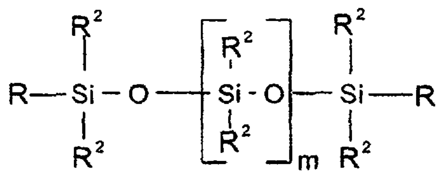

(II)

dans laquelle : R = $-(CH_2)_s\,O-(C_2H_4O)_t\,(C_3H_6O)_uR^1$
où :

- $R^1$ représente H, $CH_3$ ou $CH_2CH_3$,
- s est un entier allant de 1 à 5, t varie de 1 à 100, u varie de 0 à 50,
- les unités ($C_2H_4O$) et ($C_3H_6O$) pouvant être réparties de façon aléatoire ou par blocs,
- les radicaux $R^2$ représentent un radical alkyle en C1-C3 ou un radical phényle,
- $5 \leq m \leq 300$,

De préférence, la silicone oxyalkylénée substituée en α-ω utilisée selon la présente invention répond à la formule générale (II) pour laquelle tous les radicaux $R^2$ sont des radicaux méthyles et :

- s va de 2 à 4,
- t va de 3 à 100,
- m va de 50 à 200.

De préférence encore, le poids moléculaire moyen de R va de 800 à 2600.

De préférence, le rapport en poids des unités $C_2H_4O$ par rapport aux unités $C_3H_6O$ va de 100:10 à 20:80. De façon avantageuse, ce rapport est d'environ 42/58.

De préférence encore, $R^1$ est le groupe méthyle.

De façon plus préférentielle encore, l'émulsion selon l'invention comprend la silicone oxyalkylénée en α-ω de formule suivante :

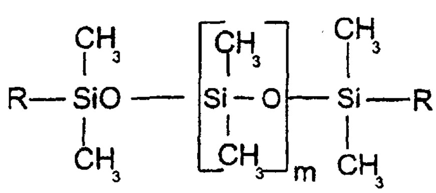

dans laquelle :

- m =100,

- R = $(CH_2)_3$-O-$(C_2H_4O)_x$-$(C_3H_6O)_y$-$CH_3$, où t va de 3 à 100, u va de 1 à 50, le rapport en poids du nombre de $C_2H_4O$ sur le nombre de $C_3H_6O$ étant d'environ 42/58, le poids moléculaire moyen de R allant de 800 à 1000.

Parmi les produits du commerce pouvant contenir tout ou partie des silicones oxyalkylénées substituées en α-ω utilisables selon l'invention comme émulsionnant, on peut citer notamment ceux vendus sous les dénominations de "Abil EM 97" par la Société Goldschmidt, ou encore de "KF 6009", "X22-4350", "X22-4349" ou "KF 6008" par la Société Shin Etsu.

L'émulsionnant polyalkyl polyéther siloxane tel que défini ci-dessus est utilisé selon l'invention en une proportion préférentielle allant de 0,1 à 30 % et plus particulièrement de 0,5 à 10 % en poids par rapport au poids total de l'émulsion.

Afin d'améliorer la persistance de la coloration sur la peau et la rémanence à l'eau de la coloration sur la peau, les compositions selon l'invention peuvent comprendre en plus au moins une dispersion aqueuse de particules de polymère filmogène (latex).

Parmi les polymères filmogènes utilisables dans le cadre de la présente invention, on peut citer les polymères synthétiques, de type radicalaire ou de type polycondensat, les polymères d'origine naturelle, et leurs mélanges.

Par polymère radicalaire, on entend un polymère obtenu par polymérisation de monomères à insaturation notamment éthylénique, chaque monomère étant susceptible de s'homopolymériser (à l'inverse des polycondensats). Les polymères de type radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont par exemple choisis parmi les esters de l'acide (méth)acrylique (encore appelé les (méth)acrylates), notamment des (méth)acrylates d'alkyle, en particulier d'alkyle en $C_1$-$C_{20}$, de préférence en $C_1$-$C_8$, des (méth)acrylates d'aryle, en particulier d'aryle en $C_6$-$C_{10}$, des (méth)acrylates d'hydroxyalkyle, en particulier d'hydroxyalkyle en $C_2$-$C_6$. Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle. Parmi les (méth)acrylates d'aryle, on peut citer l'acrylate de benzyle et l'acrylate de phényle. Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide et le N-t-octyl acrylamide.

Les polymères vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le néodécanoate de vinyle, le pivalate de vinyle, le benzoate de vinyle et le t-butyl benzoate de vinyle. Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère acrylique utilisable selon l'invention, on peut citer ceux vendus sous les dénominations NEOCRYL XK-90®, NEOCRYL A-1070®, NEOCRYL BT-62®, NEOCRYL A-1079®, NEOCRYL A-523® par la société ZENECA, DOW LATEX 432® par la société DOW CHEMICAL. On utilisera plus particulièrement le terpolymère acétate de vinyle/p-tertio-buty) benzoate de vinyle/acide crotonique (65/25/10) en dispersion aqueuse à 21% en poids.

On peut citer, parmi les polycondensats, les polyuréthannes anioniques, cationiques, non-ioniques ou amphotères, les polyuréthannes-acryliques, les poly-uréthannes-polyvinylpyrrolidones, les polyester-polyuréthannes, les polyéther-polyuréthannes, les polyurées, les polyuréepolyurée/polyuréthannes, et leurs mélanges.

Le polyuréthanne peut être, par exemple, un copolymère polyuréthanne, polyurée/uréthanne, ou polyurée, aliphatique, cycloaliphatique ou aromatique, comportant seul ou en mélange :

- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire. Comme polyuréthanne utilisable selon l'invention on peut utiliser ceux commercialisés sous les dénominations NEOREZ R-981®, NEOREZ R-974® par la société ZENECA, les SANCURE 815®, SANCURE 875®, SANCURE 2060®, SANCURE 2255®, SANCURE 861® par la société SANNCOR.

Parmi les polycondensats, on peut également citer les polyesters, les polyesters amides, les polyesters à chaîne grasse, les polyamides, et les résines époxyesters.

Les polyesters peuvent être obtenus, de façon connue, par polycondensation d'acides dicarboxyliques avec des polyols, notamment des diols. L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$: l'acide sulfoisophtalique, l'acide sulfotéréphtalique, l'acide sulfophtalique, l'acide 4-sulfonaphtalène-2,7-dicarboxylique.

On peut utiliser en particulier des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion comprenant un ou plusieurs polymères filmogènes peut être préparée par l'homme du métier sur base de ses connaissances générales. La taille des particules de polymères en dispersion aqueuse peut aller de 10 à 500 nm, et est de préférence de 20 à 150 nm.

Le polymère filmogène en dispersion aqueuse peut être présent dans les compositions selon l'invention en une teneur allant de 1 % à 60 % en poids, de préférence de 5 % à 40% en poids de matière sèche de polymères filmogènes par rapport au poids total de la composition.

Les compositions cosmétiques et/ou dermatologiques visées par la présente invention peuvent contenir un ou plusieurs solvants organiques comme par exemple un monoalcool inférieur en $C_2$-$C_6$ tel que l'éthanol ou l'isopropanol ; un polyol tel que le glycérol, le butylèneglycol, l'isoprèneglycol, le propylèneglycol.

Les compositions cosmétiques et/ou dermatologiques visées par la présente invention peuvent contenir en plus un ou plusieurs filtres solaires actifs dans l'UVA et/ou l'UVB (absorbeurs), hydrophiles ou lipophiles. Ces filtres peuvent être notamment choisis parmi les dérivés cinnamiques, les dérivés salicyliques, les dérivés du dibenzoylméthane, les dérivés du benzylidène camphre, les dérivés de benzimidazole, les dérivés de triazine, les dérivés de la benzophénone, les dérivés de β, β'-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0487404.

Les compositions cosmétiques et/ou dermatologiques selon l'invention peuvent encore contenir des pigments ou bien encore des nanopigments d'oxydes métalliques, enrobés ou non, autres que ceux de l'invention comme par exemple des nanopigments d'oxyde de titane (amorphe ou cristallisé sous forme rutile et/ou anatase), de zinc, de zirconium ou de cérium. Des agents d'enrobage classiques sont par ailleurs l'alumine et/ou le stéarate d'aluminium, les silicones. De tels nanopigments d'oxydes métalliques, enrobés ou non enrobés, sont en particulier décrits dans les demande de brevets EP-A- 0 518 772 et EP-A-0518 773.

Les compositions selon l'invention peuvent également contenir des épaississants qui peuvent être choisis notamment parmi les acides polyacryliques réticulés ; les acides polyacryliques à chaîne grasse ; les gommes de guar et celluloses modifiées ou non telles que la gomme de guar hydroxypropylée, la méthylhydroxyéthylcellulose et l'hydroxypropylméthyl cellulose ; les argiles modifiées telles que le silicate de magnésium modifié (bentone gel VS38 de RHEOX), l'hectorite modifiée par le chlorure de distéaryl diméthyl ammonium (bentone 38 CE de RHEOX).

Les compositions selon l'invention peuvent également comprendre des charges habituellement utilisées dans les compositions cosmétiques. Les charges, qui peuvent être utilisées préférentiellement peuvent être minérales ou de synthèse, lamellaires ou non lamellaires. On peut citer le talc, le mica, la silice, le kaolin, le Téflon, l'amidon, la nacre naturelle, le nitrure de bore, les microsphères telles que l'Expancel (Nobel Industrie), les microéponges telles que le polytrap (Dow Corning). De préférence, on utilise des charges sphériques dont la taille est inférieure à 25 μm telles que les poudres de polyéthylène, les poudres de Nylon, les microbilles de résine de silicone (Tospearls de Toshiba), les microsphères de silice.

Les compositions conformes à la présente invention peuvent comprendre en outre des adjuvants cosmétiques et/ou dermatologiques classiques notamment choisis parmi les adoucissants, les antioxydants, les agents anti radicaux libres, les opacifiants, les stabilisants, les émollients, les hydroxyacides, les agents anti-mousse, les agents hydratants, les vitamines, les céramides, les parfums, les conservateurs, les tensioactifs, les séquestrants, les polymères, les propulseurs, les agents alcalinisants ou acidifiants, les colorants, ou tout autre ingrédient habituellement utilisé dans les émulsions eau-dans-huile pour la coloration artificielle de la peau.

Bien entendu, l'homme de l'art veillera à choisir le ou les éventuels composés complémentaires cités ci-dessus et/ou leurs quantités de manière telle que les propriétés avantageuses attachées intrinsèquement aux émulsions eau-dans-huile à base de nanopigments d'oxyde de fer conformes à l'invention ne soient pas, ou substantiellement pas, altérées par la ou les adjonctions envisagées.

Les compositions conformes à l'invention peuvent se présenter sous forme de crèmes, de laits, de gels, de gel-crèmes, de lotions fluides, de lotions fluides vaporisables, ou tout autre forme généralement utilisée en cosmétique, en particulier celle convenant habituellement aux compositions cosmétiques pour la coloration artificielle de la peau.

Les compositions selon l'invention peuvent être préparées selon les techniques bien connues de l'homme de l'art, en particulier celles destinées à la préparation d'émulsions de type eau-dans-huile.

La présente invention a encore pour objet l'utilisation d'une émulsion eau-dans-huile telle que définie précédemment contenant au moins un nanopigment d'oxyde de fer dans des, ou pour la fabrication de, compositions cosmétiques et/ou dermatologiques destinées à conférer à la peau une coloration artificielle proche du bronzage naturel.

La présente invention a également pour objet un procédé de coloration artificielle proche du bronzage naturel, caractérisé en ce qu'il consiste à appliquer sur celle-ci une quantité efficace d'une composition cosmétique telle que définie ci-dessus.

## EXEMPLE 1

Cet exemple vise à montrer, dans un premier temps, par une méthode d'évaluation in vivo, l'intensité, la persistance et la rémanence à l'eau de la coloration obtenue avec une composition A sous forme d'émulsion eau-dans-huile contenant des nanopigments d'oxyde de fer ainsi que la rapidité avec laquelle cette coloration se développe.

Dans un deuxième temps, par une méthode d'évaluation in vitro, on a montré l'amélioration, en terme de transparence et de pouvoir colorant apportée par ladite composition A par rapport à une composition B classique de l'art antérieur sous forme d'émulsion eau-dans-huile contenant des pigments d'oxydes de fer.

La Demanderesse a réalisé la composition A suivante :

**Composition A:**

- Silicone oxyalkylénée substituées en α-ω vendue sous le nom "Abil EM 97" par la Société Goldschmidt 6,0 g
- Isostéaryl diglycéryl succinate vendu sous la dénomination commerciale "Imwitor 780 K" par la Société Hüls 2,0g
- P-hydroxybenzoate de butyle 0,015 g
- Mélange polydiphényl diméthylsiloxane/cyclopentadiméthylsiloxane vendu sous le nom MIRASIL C-DPDM par la Société RHODIA CHIMIE 8,0 g
- Cyclométhicone vendu sous le nom DC245 Fluid par la Société DOW CORNING 14,0 g
- Hectorite modifiée 4,0 g
- Isododécane 5,0 g
- Poudre de nylon 8,0 g
- Nanooxyde de fer rouge enrobé (31nm) en dispersion à 40% dans le DC345 vendu sous le nom «Nanoguard WCD 2015 » par la Société NANOPHASE TECHNOLOGIES 3,0g
- Nanooxyde de fer noir (23nm) vendu sous le nom «Nanoguard FE45BL» par la Société NANOPHASE TECHNOLOGIES 0,3 g
- Alcool éthylique 5,0 g
- Sulfate de magnésium 0,7 g
- Adipate de diisopropyle 1,0 g
- Copolymère acétate de vinyle/p-tertio-butyl-benzoate de vinyle/acide crotonique (65/25/10) en dispersion aqueuse à 21% partiellement neutralisé, stabilisé 20,0 g
- Conservateur 0,25 g
- Eau déminéralisée 22,73g

**I/Méthode d'évaluation in vivo:**

La composition A a été appliquée à raison de 2 mg/cm$^2$ sur une zone de 2,5 x 2,5 cm$^2$ délimitée sur le dos del'avant-bras de 4 volontaires.

Les six séries de mesures colorimétriques suivantes ont été effectuées à l'aide d'un colorimètre Minolta CM-1000 :

- 1°) avant application de la composition ($T_0$),
- 2°) 15 minutes après l'application ($T_{15min}$);
- 3°) 1 heures après application ($T_{1h}$);
- 3°) 3 heures après application ($T_{3h}$);
- 3°) 5 heures après application ($T_{5h}$);
- 3°) 24 heures après application ($T_{24h}$);
- 3°) 24 heures après application et après lavage ($T_{24H}$ lavage).

Les résultats sous forme de moyennes sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

Pour l'évaluation de l'intensité de la coloration, on s'intéresse

- au $\Delta L^* = L^*(T) - L^*(T_0)$ qui traduit l'assombrissement de la couleur : plus le AL* est négatif, plus la couleur s'est assombrie,
- aux $\Delta a^*$ et $\Delta b^*$ correspondants ;
- aux rapports $\Delta a^*/\Delta b^*$ correspondants qui traduisent l'équilibre rouge/jaune et donc la nuance : plus $\Delta a^*/\Delta b^*$ est proche de 1, plus la nuance est naturelle.

Les résultats obtenus sont rassemblés dans le tableau (I) suivant :

Tableau (I):

| Temps écoulé après application de la composition A | $\Delta L^* = L^*(T) - L^*(T_0)$ | $\Delta a^*/\Delta b^*$ |
|---|---|---|
| 15mn | -10,43 | 0,82 |
| 1h | -9,12 | 0,79 |
| 3h | -8,87 | 0,82 |
| 5h | -8,68 | 0,9 |
| 24h | -3,94 | 0,79 |
| 24h avec lavage | -1,52 | 0,88 |

La valeur de **ΔL*** obtenue 15 minutes après application de la composition A selon l'invention est déjà -10,43 ; ce qui démontre un assombrissement rapide de la peau et de bonne intensité.

Les valeurs de **ΔL*** obtenue 24 heures après application de la composition A selon l'invention sont de -3,94 (sans lavage) et -1,52 (avec lavage) ; ce qui traduit une coloration sur la peau persistante dans le temps et résistante à l'eau.

Les rapports **Δa*/Δb*** mesurés après 15mn, 1h, 3h, 5h, 24h et 24h après lavage, varient de 0,82 à 0,9 ; ces résultats montrent que la coloration obtenue présente une nuance bien équilibrée, proche de la coloration naturelle de la peau , constante dans le temps et résistante à l'eau.

**II/Méthode d'évaluation in vitro**

La Demanderesse a également réalisé une composition B ( ne faisant pas partie de l'invention) identique à la composition A mais contenant à la place des nanopigments d'oxyde de fer :

- 3g d'oxyde de fer noir pigmentaire (2.7μm) vendu sous le nom « COSMETIC BLACK C33-5198 (SUN) » par la Société SUN ; et
- 0,3 g d'oxyde de fer brun pigmentaire (1.9 μm) vendu sous le nom « SICOMET brun ZP3569 » par la société BASF.

Pour évaluer la transparence et le pouvoir colorant apportée par chacune des formulations A et B, on a mis en oeuvre la méthode de caractérisation utilisée dans l'industrie des peintures et décrite dans l'ouvrage « Industrial color testing » de Hans G. Völz, pages 101-111; Edition VCH.

On étale sur une plaque de contraste normalisée possédant une partie noire et une partie blanche calibrées colorimétriquement, des films de différentes épaisseur formés à partir de chaque composition testée A et B : 12, 30, 60 μm.

Les mesures colorimétriques suivantes ont été effectuées à l'aide d'un colorimètre Minolta CM-1000

- 1 °) avant application de la composition ($T_0$),
- 2°) après l'application après 15 mn ($T_{15mn}$);

Les résultats sont exprimés dans le système ($L^*$, $a^*$, $b^*$) dans lequel $L^*$ représente la luminance, $a^*$ représente l'axe rouge-vert (-a = vert, +a = rouge) et $b^*$ représente l'axe jaune-bleu (-$b^*$ =bleu, +$b^*$ =jaune). Ainsi, $a^*$ et $b^*$ expriment

la nuance du film sur la plaque.

Pour l'évaluation de la transparence ou du pouvoir colorant du film appliqué, on s'intéresse :

- à $\Delta L^* = L(T_{15mn}) - L(T_0)$
- aux $\Delta a^*$ et $\Delta b^*$ correspondants,
- au $\Delta E^*_{ab} = \sqrt{\Delta a^{*2} + \Delta b^{*2} + \Delta L^{*2}}$

1°) *Transparence*

La transparence est caractérisée par la variation de $\Delta E^*_{ab}$ en fonction de l'épaisseur du film, lorsque le $\Delta E^*_{ab}$ est mesuré sur fond noir. Plus la valeur du $\Delta E^*_{ab}$ est élevée, plus le produit appliqué est opacifiant.

Les résultats obtenus sont rassemblés dans le tableau (II) suivant :

| COMPOSITION A (invention) | | | | |
|---|---|---|---|---|
| **épaisseur** | **ΔL*** | **Δa*** | **Δb*** | **$\Delta E_{ab}$*** |
| 12 µm | 14.393 | 1.757 | 6.453 | 15.871 |
| 30 µm | 16.723 | 3.657 | 8.793 | 19.245 |
| 60 µm | 19.083 | 7.027 | 12.573 | 23.909 |
| COMPOSITION B(hors invention) | | | | |
| **épaisseur** | **ΔL** | **Δa*** | **Δb*** | **$\Delta E_{ab}$*** |
| 12 µm | 22.363 | 13.647 | 11.223 | 28.501 |
| 30 µm | 25.443 | 18.197 | 15.203 | 34.780 |
| 60 µm | 25.823 | 19.617 | 16.263 | 36.279 |

On constate:

- que la composition B appliquée sur le substrat noir à raison d'une couche de 12µm est déjà, à cette quantité, 2 fois plus opacifiante que la composition A de l'invention appliquée dans les mêmes conditions ;
- que la variation de $\Delta E_{ab}$* en fonction de l'épaisseur du film est beaucoup plus importante avec la composition B (oxyde de fer de taille micrométrique), et que celle-ci s'avère plus couvrante, plus apte à opacifier le substrat sur lequel elle est déposée que la composition A selon l'invention contenant les oxydes de fer de taille nanométrique.

2°) *Pouvoir colorant*

Le pouvoir colorant est caractérisé par la variation du $\Delta E_{ab}$* en fonction de l'épaisseur du film, lorsque $\Delta E_{ab}$* est mesuré sur fond blanc. Plus la valeur du $\Delta E_{ab}$* est élevée, plus le film a tendance à colorer la plaque.

Les résultats obtenus sont rassemblés dans le tableau (III) suivant:

Tableau (III)

| COMPOSITION A (invention) | | | | |
|---|---|---|---|---|
| **épaisseur** | **ΔL** | **Δa** | **Δb** | **$\Delta E_{ab}$*** |
| 12 µm | -22.56 | 22.793 | 40.753 | 51.86 |
| 30 µm | -32.69 | 30.193 | 44.043 | 62.61 |
| 60 µm | -40.84 | 34.233 | 40.453 | 66.9 |
| COMPOSITION B (hors invention) | | | | |
| **épaisseur** | **ΔL** | Δa | **Δb** | **$\Delta E_{ab}$*** |
| 12 µm | -14.56 | 14.283 | 12.103 | 23.717 |
| 30 µm | -42.27 | 25.293 | 18.313 | 52.554 |
| 60 µm | -47.60 | 25.353 | 17.593 | 56.728 |

On constate :

- que la composition A de l'invention appliquée sur le substrat blanc à raison d'une couche de 12µm est déjà, à cette quantité, environ 2 fois plus colorante que la composition B appliquée dans les mêmes conditions
- que la variation de $AE_{ab}^*$en fonction de l'épaisseur est beaucoup plus rapide et importante avec la composition A (oxyde de fer de taille nanométrique) et présente donc un pouvoir teintant plus important que la composition B contenant des oxydes de fer de taille micrométrique.

## Revendications

1. Composition cosmétique et/ou dermatologique, destinée à produire sur la peau une coloration artificielle proche du bronzage naturel, **caractérisée par le fait qu'**elle comprend, dans un support sous forme d'émulsion eau-dans-huile, plus de 2% en poids par rapport au poids de ladite composition, d'au moins un nanopigment d'oxyde de fer dont la taille moyenne des particules élémentaires est inférieure à 100nm.

2. Composition selon la revendication 1, où la taille moyenne des particules élémentaires d'oxyde de fer varie de 5 nm à 50 nm, et plus particulièrement de 10 à 30 nm.

3. Composition selon la revendication 1 ou 2, où le nanopigment d'oxyde de fer est présent dans des proportions allant jusqu'à 10% en poids et plus particulièrement variant de 3 à 6% en poids par rapport au poids total de la composition.

4. Composition selon l'une quelconque des revendications 1 à 3, où le support est une émulsion eau-dans-silicone dans laquelle la phase huileuse continue comprend au moins une huile de silicone.

5. Composition selon la revendication 4, où l'huile de silicone est volatile.

6. Composition selon la revendication 4 ou 5, où la phase continue huileuse contient au moins une huile siliconée choisie parmi les polydiorganosiloxanes linéaires, éventuellement fonctionnalisés, ou les polydiorganosiloxanes cycliques ou les organopolysiloxanes éventuellement réticulés ou les mélanges de ceux-ci.

7. Composition selon la revendication 6, où les polydiorganosiloxanes linéaires répondent à la structure suivante :

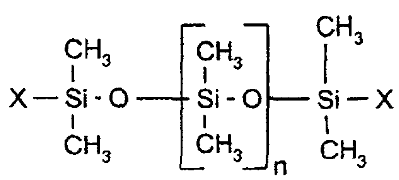

dans laquelle :

- X est $-CH_3$ ou OH, et
- n est un entier allant de 0 à 2000.

8. Composition selon la revendication 6, où les polydiorganosiloxanes cycliques répondent à la formule suivante :

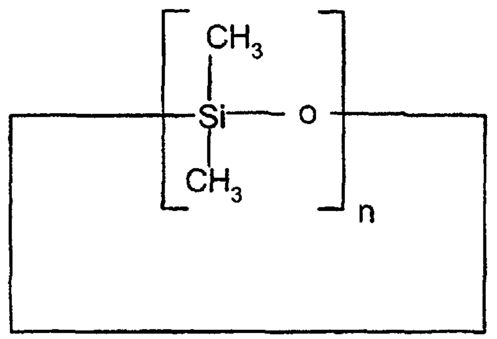

dans laquelle n est un nombre entier de 3 à 8.

9. Composition selon l'une quelconque des revendications 1 à 8, comprenant au moins un émulsionnant siliconé constitué d'un polyalkyl polyéther siloxane portant des chaînes polyoxyéthylènes et polyoxypropylènes greffées sur la chaîne principale ou aux extrémités de la chaîne principale..

10. Composition selon la revendication 9, où l'émulsionnant siliconé est choisi parmi les composés de formule générale (I) suivante :

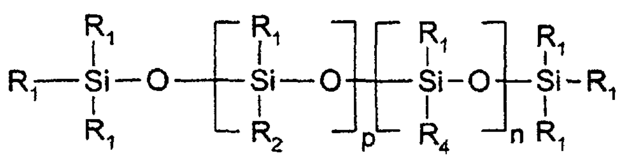 (I)

formule dans laquelle :

- $R_1$ et $R_4$, identiques ou différents, représentent un atome d'hydrogène, un radical alkyle linéaire ou ramifié en $C_1$-$C_{30}$ ou un radical phényle,
- $R_2$, identiques ou différents, représentent $-(C_xH_{2x})-(OC_2H_4)_a-(OC_3H_6)_b-OR_3$,
- $R_3$, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 1 à 1000,
- p varie de 1 à 30,
- a varie de 1 à 50,
- b varie de 1 à 50,
- x varie de 1 à 5.

11. Composition selon la revendication 10, **caractérisée par le fait que** les radicaux R, et $R_4$ sont identiques et représentent tous des radicaux méthyle et le radical $R_3$ représente l'hydrogène.

12. Composition selon la revendication 11, **caractérisée par le fait que** ledit polyalky polyéther siloxane est un poly diméthyl/méthyl siloxane oxyéthyléné oxypropyléné de poids moléculaire en nombre supérieur à 30 000 et pour lequel n est 396, p est 4, a est 18 et b est 18.

13. Composition selon la revendication 10, **caractérisée par le fait que** les radicaux $R_1$ représentent tous des radicaux méthyle et les radicaux $R_4$ représentent tous des radicaux lauryle.

14. Composition selon la revendication 13, **caractérisée par le fait que** ledit polyalky polyéther siloxane est un poly méthyllauryl/méthyl siloxane oxyéthyléné oxypropyléné de poids moléculaire en nombre supérieur à 25000 et pour lequel n est 35, p est 3, a est 18 et b est 18.

15. Composition selon la revendication 9, **caractérisée par le fait que** l'émulsionnant siliconé est une silicone oxyalkylénée substituée en $\alpha$-$\omega$ à structure linéaire, substitué aux deux extrémités de la chaîne principale par des groupements oxyalkylène reliés aux atomes de Si par l'intermédiaire d'un groupement hydrocarboné.

**16.** Composition selon la revendication 15, où l'émulsionnant siliconé répond à la formule générale (II) suivante

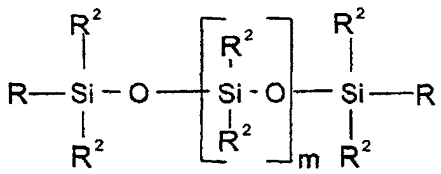

(II)

dans laquelle : R = $-(CH_2)_s\ O-(C_2H_4O)_t\ (C_3H_6O)_uR^1$ où : - $R^1$ représente H, $CH_3$ ou $CH_2CH_3$,

- s est un entier allant de 1 à 5, t varie de 1 à 100, u varie de 0 à 50,
- les unités $(C_2H_4O)$ et $(C_3H_6O)$ pouvant être réparties de façon aléatoire ou par blocs,
- les radicaux $R^2$ représentent un radical alkyle en C1-C3 ou un radical phényle,
- $5 \leq m \leq 300$,

**17.** Composition selon la revendication 16, où tous les radicaux $R^2$ sont des radicaux méthyles et s varie de 2 à 4, t varie de 3 à 100 et m varie de 50 à 200.

**18.** Composition selon la revendication 16 ou 17, où le poids moléculaire moyen de R varie de 800 à 2600 et où le rapport en poids des unités $C_2H_4O$ par rapport aux unités $C_3H_6O$ varie de 100:10 à 20:80.

**19.** Composition selon la revendication 16, où la silicone oxyalkylénée en $\alpha$-$\omega$ est de formule suivante :

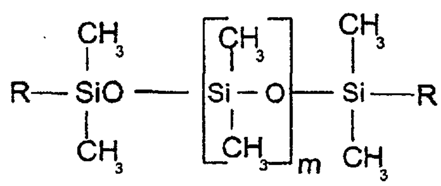

dans laquelle :

- m = 100,
- R = $(CH_2)_3-O-(C_2H_4O)_x-(C_3H_6O)_y-CH_3$, où t va de 3 à 100, u va de 1 à 50, le rapport en poids du nombre de $C_2H_4O$ sur le nombre de $C_3H_6O$ est d'environ 42/58, le poids moléculaire moyen de R varie de 800 à 1000.

**20.** Composition selon l'une quelconque des revendications 9 à 19, où l'émulsionnant polyalkyl polyéther siloxane est utilisé selon l'invention en une proportion allant de 0,1 à 30 % et plus particulièrement de 0,5 à 10 % en poids par rapport au poids total de la composition.

**21.** Composition selon l'une quelconque des revendications 1 à 19, contenant en plus au moins une dispersion aqueuse de particules de polymère filmogène (latex).

**22.** Utilisation d'une émulsion eau-dans-huile telle que définie dans l'une quelconque des revendications 1 à 21 contenant au moins un nanopigment d'oxyde de fer dans des compositions cosmétiques et/ou pour la fabrication de compositions dermatologiques, pour produire sur la peau une coloration artificielle proche du bronzage naturel.

**23.** Procédé cosmétique de coloration artificielle de la peau proche du bronzage naturel, **caractérisé en ce qu'**il consiste à appliquer sur celle-ci une quantité efficace d'une composition cosmétique telle que définie dans l'une quelconque des revendications 1 à 21.

**Patentansprüche**

1. Kosmetische und/oder dermatologische Zusammensetzung, die dazu vorgesehen ist, auf der Haut eine künstliche Färbung zu erzeugen, die der natürlichen Bräune nahe kommt, **dadurch gekennzeichnet, dass** sie in einem als Wasser-in-Öl-Emulsion vorliegenden Träger über 2 Gew.-% mindestens eines Eisenoxid-Nanopigments mit einer mittleren Größe der Elementarpartikel unter 100 nm, bezogen auf das Gesamtgewicht der Zusammensetzung, enthält.

2. Zusammensetzung nach Anspruch 1, wobei die mittlere Größe der Elementarpartikel des Eisenoxids im Bereich von 5 bis 50 nm und insbesondere 10 bis 30 nm liegt.

3. Zusammensetzung nach Anspruch 1 oder 2, wobei das Eisenoxid-Nanopigment in Mengenanteilen von bis zu 10 Gew.-% und insbesondere im Bereich von 3 bis 6 Gew.-%, bezogen auf das Gesamtgewicht der Zusammensetzung, vorliegt.

4. Zusammensetzung nach einem der Ansprüche 1 bis 3, wobei der Träger eine Wasser-in-Silicon-Emulsion ist, deren kontinuierliche Ölphase mindestens ein Siliconöl enthält.

5. Zusammensetzung nach Anspruch 4, wobei das Siliconöl flüchtig ist.

6. Zusammensetzung nach Anspruch 4 oder 5, wobei die kontinuierliche Ölphase mindestens ein Siliconöl enthalt, das unter den geradkettigen, gegebenenfalls funktionalisierten Polydiorganosiloxanen, den cyclischen Polydiorganosiloxanen oder den gegebenenfalls vernetzten Organopolysiloxanen oder deren Gemischen ausgewählt ist.

7. Zusammensetzung nach Anspruch 6, wobei die geradkettigen Polydiorganosiloxane der folgenden Struktur entsprechen:

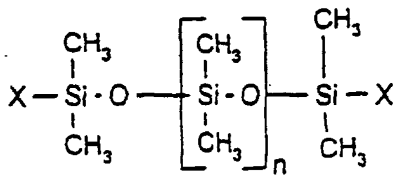

worin bedeuten:

- X $-CH_3$ oder OH, und
- n 0 oder eine ganze Zahl von 1 bis 2000.

8. Zusammensetzung nach Anspruch 6, wobei die cyclischen Polydiorganosiloxane der folgenden Struktur entsprechen:

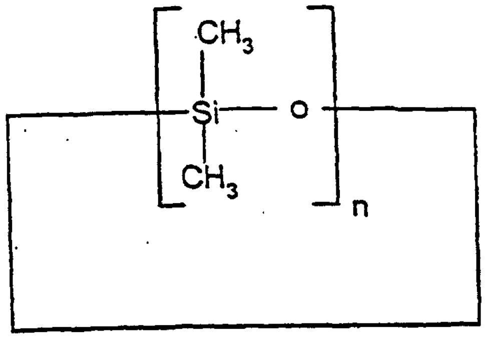

worin n eine ganze Zahl von 3 bis 8 bedeutet.

**9.** Zusammensetzung nach einem der Ansprüche 1 bis 8, die mindestens einen Siliconemulgator enthält, der aus einem Polyalkylpolyethersiloxan besteht, das auf die Hauptkette gepfropft oder an den Enden polyethoxylierte und polypropoxylierte Gruppen trägt.

**10.** Zusammensetzung nach Anspruch 9, wobei der Siliconemulgator unter den Verbindungen der folgenden allgemeinen Formel (I) ausgewählt ist:

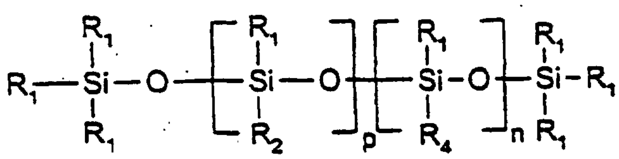

(I),

worin:

- die Gruppen $R_1$ und $R_4$, die identisch oder voneinander verschieden sind, ein Wasserstoffatom, eine geradkettige oder verzweigte $C_{1-30}$-Alkylgruppe oder eine Phenylgruppe bedeuten,
- die Gruppen $R_2$, die identisch oder voneinander verschieden sind, - $(C_xH_{2x})$-$(OC_2H_4)a$-$(OC_3H_6)_b$-$OR_3$ bedeuten,
- die Gruppen $R_3$, die identisch oder voneinander verschieden sind, unter Wasserstoff, einer geradkettigen oder verzweigten Alkylgruppe mit 1 bis 12 Kohlenstoffatomen oder einer geradkettigen oder verzweigten Acylgruppe mit 2 bis 12 Kohlenstoffatomen ausgewählt sind,
- n im Bereich von 1 bis 1000 liegt,
- p im Bereich von 1 bis 30 liegt,
- a im Bereich von 1 bis 50 liegt,
- b im Bereich von 1 bis 50 liegt, und
- x im Bereich von 1 bis 5 liegt.

**11.** Zusammensetzung nach Anspruch 10, **dadurch gekennzeichnet, dass** die Gruppen $R_1$ und $R_4$ identisch sind und Methylgruppen bedeuten und die Gruppe $R_3$ Wasserstoff bedeutet.

**12.** Zusammensetzung nach Anspruch 11, wobei das Polyalkylpolyethersiloxan ein ethoxyliertes und propoxyliertes Polydimethyl/methylsiloxan mit einem Zahlenmittel des Molekulargewichts über 30 000 ist, wobei n 396, p 4, a 18 und b 18 bedeutet.

**13.** Zusammensetzung nach Anspruch 10, **dadurch gekennzeichnet, dass** die Gruppen $R_1$ alle Methylgruppen und die Gruppen $R_4$ alle Laurylgruppen sind.

**14.** Zusammensetzung nach Anspruch 13, **dadurch gekennzeichnet, dass** das Polyalkylpolyethersiloxan ein ethoxyliertes und propoxyliertes Polymethyllauryl/methylsiloxan mit einem Zahlenmittel des Molekulargewichts über 25 000 ist, wobei n 35 und p 3, a 18 und b 18 bedeutet.

**15.** Zusammensetzung nach Anspruch 9, **dadurch gekennzeichnet, dass** der Siliconemulgator ein in $\alpha$-$\omega$-Stellung substituiertes, alkoxyliertes Silicon mit gerader Struktur ist, das an beiden Enden der Hauptkette mit Alkylenoxidgruppen substituiert ist, die über eine Kohlenwasserstoffgruppe an die Siliciumatome gebunden sind.

**16.** Zusammensetzung nach Anspruch 15, wobei der Siliconemulgator der folgenden allgemeinen Formel (II) entspricht:

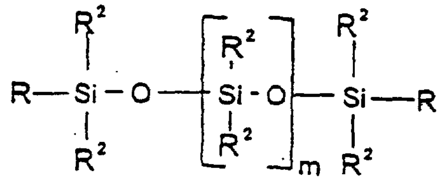

(II),

worin R $-(CH_2)sO-(C_2H_4O)t-(C_3H_6O)uR^1$ bedeutet, mit:

- $R^1$ bedeutet H, $CH_3$ oder $CH_2CH_3$;
- s ist eine ganze Zahl von 1 bis 5, t liegt im Bereich von 1 bis 100 und u liegt im Bereich von 0 bis 50;
- die Einheiten ($C_2H_4O$) und ($C_3H_6O$) können zufällig verteilt oder als Blöcke vorliegen;

die Gruppen $R^2$ eine $C_{1-3}$-Alkylgruppe oder Phenyl bedeuten, und
$5 \leq m \leq 300$.

**17.** Zusammensetzung nach Anspruch 16, wobei alle Gruppen $R^2$ Methyl bedeuten und s im Bereich von 2 bis 4 liegt, t im Bereich von 3 bis 100 liegt und m im Bereich von 50 bis 200 liegt.

**18.** Zusammensetzung nach Anspruch 16 oder 17, wobei die mittlere Molmasse von R im Bereich von 800 bis 2600 und/oder das Gewichtsverhältnis der Einheiten $C_2H_4O$ und der Einheiten $C_3H_6O$ im Bereich von 100:10 bis 20:80 liegt.

**19.** Zusammensetzung nach Anspruch 16, wobei das in $\alpha$-$\omega$-Stellung substituierte, alkoxylierte Silicon der folgenden Formel entspricht:

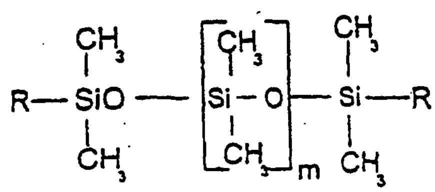

worin bedeuten:
m = 100,
R = $-(CH_2)_3O-(C_2H_4O)_x-(C_3H_6O)_y-CH_3$, wobei t im Bereich von 3 bis 100 liegt, u im Bereich von 1 bis 50 liegt, das Gewichtsverhältnis der Einheiten $C_2H_4O$ und der Einheiten $C_3H_6O$ etwa 42/58 beträgt und die mittlere Molmasse von R im Bereich von 800 bis 1000 liegt.

**20.** Zusammensetzung nach einem der Ansprüche 9 bis 19, wobei das emulgierende Polyalkylpolyethersiloxan erfindungsgemäß in Konzentrationen von 0,1 bis 30 % und insbesondere 0,5 bis 10 Gew.-%, bezogen auf das Gesamtgewicht der Zusammensetzung, verwendet wird.

**21.** Zusammensetzung nach einem der Ansprüche 1 bis 19, die ferner mindestens eine wässrige Dispersion von Partikeln eines filmbildenden Polymers (Latex) enthält.

**22.** Verwendung einer Wasser-in-Öl-Emulsion nach einem der Ansprüche 1 bis 21, die mindestens ein Eisenoxid-Nanopigment enthält, in kosmetischen Zusammensetzungen und/oder zur Herstellung von dermatologischen Zusammensetzungen, um der Haut eine künstliche Färbung ähnlich der natürlichen Bräune zu geben.

**23.** Kosmetisches Verfahren zur künstlichen Färbung der Haut ähnlich der natürlichen Bräune, **dadurch gekennzeichnet, dass** eine wirksame Menge einer kosmetischen Zusammensetzung nach einem der Ansprüche 1 bis 21 auf die Haut aufgebracht wird.

**Claims**

**1.** Cosmetic and/or dermatological formulation, intended to produce an artificial colour similar to a natural tan on the skin, **characterised in that** it comprises, in a water-in-oil emulsion form substrate, over 2 % by weight with respect to the weight of said formulation consisting of at least one iron oxide nanopigment wherein the average elementary particle size is less than 100 nm.

**2.** Formulation according to claim 1, wherein the mean elementary iron oxide particle size varies from 5 nm to 50 nm, and more particularly from 10 to 30 nm.

**3.** Formulation according to claim 1 or 2, wherein the iron oxide nanopigment is present in proportions of up to 10 % by weight and more particularly varying from 3 to 6 % by weight with respect to the total weight of the formulation.

**4.** Formulation according to any of claims 1 to 3, wherein the substrate is a water-in-silicone emulsion wherein the continuous oily phase comprises at least one silicone oil.

**5.** Formulation according to claim 4, wherein the silicone oil is volatile.

**6.** Formulation according to claim 4 or 5, wherein the continuous oily phase contains at least one silicone oil selected from linear, functionalised if applicable, or cyclic polydiorganosiloxanes or organopolysiloxanes, cross-linked if applicable, or mixtures thereof.

**7.** Formulation according to claim 6, wherein the linear polydiorganosiloxanes comply with the following structure :

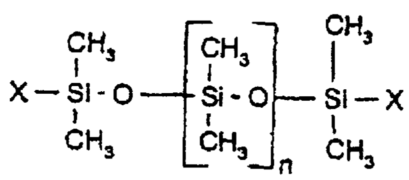

wherein :

- X is $-CH_3$ or OH, and
- n is an integer between 0 and 2000.

**8.** Formulation according to claim 6, wherein the cyclic polydiorganosiloxanes comply with the following formula :

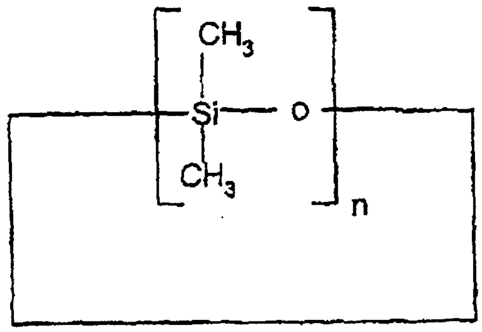

wherein n is an integer from 3 to 8.

**9.** Formulation according to any of claims 1 to 8, comprising at least one silicone emulsifier consisting of a polyalkyl polyether siloxane comprising polyoxyethylene and polyoxypropylene chains grafted onto the main chain or at the end of the main chain.

**10.** Formulation according to claim 9, wherein the silicon emulsifier is selected from the compounds according to the following general formula I :

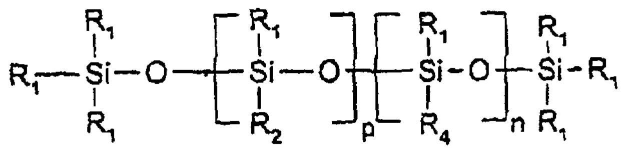 (I)

wherein :

- $R_1$ and $R_4$, identical or different, represent a hydrogen atom, a linear or ramified $C_1$-$C_{30}$ alkyl radical or a phenyl radical,
- $R_2$, identical or different, represent $-(C_xH_{2x})-(OC_2H_4-(OC_3H_6)_b-OR_3$ ,
- $R_3$, identical or different, are selected from a hydrogen atom, a linear or ramified alkyl radical, having 1 to 12 carbon atoms, a linear or ramified acyl radical having 2 to 12 carbon atoms,
- n varies from 1 to 1000,
- p varies from 1 to 30,
- a varies from 1 to 50,
- b varies from 1 to 50,
- x varies from 1 to 5.

**11.** Formulation according to claim 10, **characterised in that** the radicals $R_1$ and $R_4$ are identical and all represent methyl radicals and the radical $R_3$ represents hydrogen.

**12.** Formulation according to claim 11, **characterised in that** said polyalky polyether siloxane is an oxyethylene oxypropylene polydimethyl/methyl siloxane of a numerical molecular weight greater than 30,000 and wherein n is 396, p is 4, a is 18 and b is 18.

**13.** Formulation according to claim 10, **characterised in that** the radicals $R_1$ all represent methyl radicals and the radicals $R_4$ all represent lauryl radicals.

**14.** Formulation according to claim 13, **characterised in that** said polyalky polyether siloxane is an oxyethylene oxypropylene polymethyllauryl/methyl siloxane of a numerical molecular weight greater than 25,000 and wherein n

is 35, p is 3, a is 18 and b is 18.

**15.** Formulation according to claim 9, **characterised in that** the silicone emulsifier is a linear structure α-ω substituted oxyalkylene silicone, substituted at both ends of the main chain by oxyalkylene groups linked to the Si atoms by a hydrocarbonate group.

**16.** Formulation according to claim 15, wherein the silicone emulsifier complies with the following general formula (II):

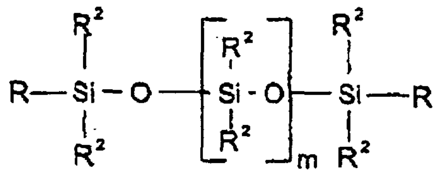

(II)

wherein : R = $-(CH_2)_s\ O-(C_2H_4O)_t\ (C_3H_6O)_uR^1$
where : - $R^1$ represents H, $CH_3$ or $CH_2CH_3$,

- s is an integer from 1 to 5, t varies from 1 to 100, u varies from 0 to 50,
- the units $(C_2H_4O)$ and $(C_3H_6O)$ can be distributed randomly or in blocks,
- the $R_2$ radicals represent a C1-C3 alkyl radical or a phenyl radical,
- 5 m 300.

**17.** Formulation according to claim 16, wherein all the $R^2$ radicals are methyl radicals and s varies from 2 to 4, t varies from 3 to 100 and m varies from 50 to 200.

**18.** Formulation according to claim 16 or 17, wherein the mean molecular weight of R varies from 800 to 2600 and wherein the ratio by weight of the $C_2H_4O$ units with respect to the $C_3H_6O$ units varies from 100 : 10 to 20:80.

**19.** Formulation according to claim 16, the α-ω oxyalkylene silicone takes the following formula :

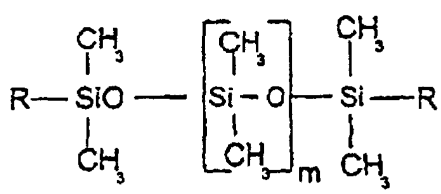

wherein:

- m = 100,
- R = $(CH_2)_3-O-(C_2H_4O)_x-(C_3H_6O)_y-CH_3$, where t is from 3 to 100, u is from 1 to 50, the ratio by weight of the number of $C_2H_4O$ over the number of $C_3H_6O$ being approximately 42/58, the mean molecular weight of R ranging from 800 to 1000.

**20.** Formulation according to any of claims 9 to 19, wherein the polyalkyl polyether siloxane emulsifier as defined above is used according to the invention in a proportion ranging from 0.1 to 30 % and more particularly from 0.5 to 10 % by weight with respect to the total weight of the emulsion.

**21.** Formulation according to any of claims 1 to 19, also containing at least one aqueous dispersion of film-forming polymer particles (latex).

**22.** Use of a water-in-oil emulsion as defined in any of claims 1 to 21 containing at least one iron oxide nanopigment

in cosmetic formulations and/or to produce dermatological formulations intended to give the skin an artificial colour similar to a natural tan.

**23.** Cosmetic artificial colouring method similar to a natural tan, **characterised in that** it consists of applying an effective quantity of a cosmetic formulation as defined in any of claims 1 to 21 onto the skin.